# FASCICULE DE BREVET EUROPEEN

(11) **EP 1 275 395 B1**
(45) Date de publication et mention de la délivrance du brevet: **18.04.2007**
(21) Numéro de dépôt: 02012527.4
(22) Date de dépôt: 05.06.2002
(51) Int. Cl.: A61K 31/728, A61K 9/02, A61P 15/02, A61K 36/886, A61K 36/28, A61K 36/61, A61K 36/23

(54) **Emploi d'acide hyaluronique pour la production d'une préparation à usage vaginal**
Verwendung von Hyaluronsäure zur Herstellung eines Präparates für vaginale Anwendung
Use of hyaluronic acid for preparing a composition for vaginal use

(30) Priorité: 07.06.2001 IT BO20010361
(43) Date de publication de la demande: 15.01.2003
(73) Titulaire: Farma-Derma S.R.L., 40010 Sala Bolognese (BO) (IT)
(72) Inventeur: Russo, Vincento, Bologne (IT)

(56) Documents cités:
- EP-A- 0 509 120
- FR-A- 2 594 690
- FR-A- 2 623 397
- FR-A- 2 662 939
- US-A- 4 670 265
- US-A- 4 708 873
- US-A- 6 159 955
- SRIVASTAVA R ET AL: "CHEMISTRY AND PHARMACOLOGY OF CENTELLA ASIATICA: A REVIEW" JOURNAL OF MEDICINAL AND AROMATIC PLANT SCIENCES, UNKNOWN, IN, vol. 19, no. 4, décembre 1997 (1997-12), pages 1049-1056, XP001032415
- HARKENTHAL M ET AL: "COMPARATIVE STUDY ON THE IN VITRO ANTIBACTERIAL ACTIVITY OF AUSTRALIAN TEA TREE OIL, CAJUPUT OIL, NIAOULI OIL, MANUKA OIL, KANUKA OIL, AND EUCALYPTUS OIL" PHARMAZIE, VEB VERLAG VOLK UND GESUNDHEIT. BERLIN, DD, vol. 54, no. 6, juin 1999 (1999-06), pages 460-463, XP001061666 ISSN: 0031-7144

## Description

L'invention concerne l'emploi d'acide hyaluronique associé à Aloès Vera, Bevilaqua Hydrocotyle Asiatique, Souci Officinal, Huile de Cajeputier pour la production d'une préparation à usage vaginal, en particulier pour le soin des états atrophiques et dystrophiques cervico-vaginaux (vaginites dues à carence d'oestrogènes, vaginites séniles, craurosis vulvo-vaginal), retard de cicatrisation cervico-vaginale du post-partum, en chirurgie gynécologique, dystrophies conséquentes à chimiothérapie ou radiations ionisantes.
Cette préparation se rapporte au secteur des produits à emploi vaginal. C'est un trophique capable de conditionner les processus physiologiques indispensables pour la réparation tissulaire.

Le rôle que la colposcopie joue dans le diagnostic des lésions de l'épithélium et dans l'infection génitale due au Papillomavirus humain (HPV) est d'importance fondamentale. On sait que la biopsie faite par colposcopie est extrêmement prévisible et offre une approche thérapeutique réalisable en ambulatoire par des procédures excisionnelles qui sont réalisables par l' anse diathermique ou laser. La facilité d'exécution et les coûts contenus ont contribué à une vaste diffusion des techniques excisionnelles électrochirurgicales qui quand même provoquent des lésions qu'on doit traiter avec une préparation qui cicatrise par deuxième intention. De plus la femme en ménopause subit des changements hormonaux qui se reflètent sur la muqueuse vaginale, qui devient moins épaisse et douce et perd l'hydratation en causant prurit et brûlure. Raison pour laquelle il est important de maintenir la muqueuse hydratée.

L'acide hyaluronique, substance produite par les fibroblastes, représente l'élément anatomo-fonctionnel principal du tissu connectif et il permet l'équilibre dynamique de ses constituants. La quantité d'acide hyaluronique dans la peau et dans les muqueuses est responsable du tonus, de la trophicité et de la souplesse de ces tissus. Dans le processus physiologique de la réparation l'acide hyaluronique représente le substrat indispensable pour la migration cellulaire. La concentration d'acide hyaluronique augmente fortement dans les zones adjacentes à la lésion jusqu'à ce que le processus de guérison soit conclu. L'administration exogène d'acide hyaluronique augmente la motilité de toutes les cellules impliquées dans le processus de réparation, avec une diminution des temps de guérison. En particulier l'acide hyaluronique augmente l'activité phagocytaire des macrophages et stimule la prolifération et migration des fibroblastes. (BREVET EP 0509 120 A1)

Le but de cette préparation est donc de créer les conditions optimales pour la migration et la prolifération des cellules responsables de la réparation tissulaire pour le soin des états atrophiques et dystrophiques cervico-vaginaux de n'importe quelle origine et nature.
Les revendications secondaires expliquent d'autres aspects avantageux de la préparation.
Les caractéristiques techniques et les différents aspects avantageux de la préparation seront plus évidents dans la lecture de la description détaillée qui suit, faite avec réference à exemples réalisables, qui représentent une forme de réalisation qui sert seulement d'exemples et n'est pas limitative.

Il est prévu d'associer, à l'acide hyaluronique, de l'Aloès Vera qui, en synergie avec le principe actif de base, fourni à la présente préparation des proprietés anti-inflammatoires ( CHITHRA P.,SAJITHLAL GB.,CHANDRAKASAN G. : Influence of Aloe Vera on the glycosaminoglycans in the matrix of heading dermal wounds in rats J. Ethnopharmacol 1998; 59(3):179-186).
En adjonction à l'Aloès Vera on utilise comme agent actif à associer à l'acide hyaluronique de la Bevilaqua Hydrocotyle Asiatique .La préparation résultante augmente les propriétés cicatrisantes, anti-inflammatoires. (MAEQUART FX., CHASTANG F., SIMEON A..et al : Triterpenes from centella asiatica stimulate extracellular matrix accumulation in rat experimental wounds. Eur. J.Dermatol.1999 ;9(4) :289-296).
Toujours en adjonction à l'Aloés Vera et à la Bevilaqua Hydrocotyle Asiatique on utilise dans la présente préparation du Souci Officinal.
On ajoute à la préparation de l'huile de cajeputier.

On obtient en définitif une préparation qui donne une activité cicatrisante, anti-inflammatoire et rééquilibrante de la muqueuse vaginale.

La partie active décrite ci dessus de la présente préparation est englobée dans un ovule approprié constitué surtout de glycérides semi-synthétiques qui se dissolvent rapidement et qui peuvent être facilement éliminés. L'application par ovule est particulièrement préférée, puisqu'il permet de maintenir le principe actif au niveau du vagin pour un temps prolongé et de le relacher progressivement.
Pour des ovules pesant entre 2 et 5 g. des résultats significatifs sont obtenus par les quantités suivantes de substances actives:

| | |
|---|---|
| Acide Hyaluronique | = de 5 à 100 mg; |
| Aloés Vera | = de 20 à 200 mg |
| Bevilaqua Hydrocotyle Asiatique | = de 20 à 200 mg. |
| Souci Officinal | = de 20 à 200 mg. |
| Huile de Cajeputier | = de 1 à 10 mg. |

Selon un exemple particulièrement préféré de la présente préparation relatif à un ovule de 2 grammes, on utilise comme principe actif un mélange qui contient les quantités suivantes de substances:

| | |
|---|---|
| Acide hyaluronique | = 5 mg. |
| Aloés Vera | = 60 mg. |
| Bevilaqua Hydrocotyle Asiatique | = 60 mg. |
| Souci Officinal | = 60 mg. |
| Huile de Cajeputier | = 2 mg. |

En outre on utilise, comme excipient, une quantité de 1810 mg. environ de glycérides semi-synthétiques pour obtenir un ovule de 2 g.
L'ovule présent est preparé en fondant, par agitation dans un thermofondeur à une température prédéfinie, les glycérides aptes à former la partie d'excipient et structurel de l'ovule.
Après un intervalle de temps prédeterminé, 30 minutes environ, on ajoute dans la masse fondue des glycérides, les composants actifs cités.
L'ordre d'adjonction prévoit de commencer par l'Huile de Cajeputier et de continuer avec l'Aloès Vera, la Bevilaqua Hydrocotyle Asiatique, le Souci Officinal et enfin l'acide hyaluronique,
le tout en gardant la masse globale toujours à la température déjà citée prédéfinie de fusion pour un intervalle ultérieur de temps d' environ 30 minutes. Dans cette phase la masse est maintenue en agitation pour procéder ensuite au remplissage des alvéoles, blister préformé à forme d'ovule apte à contenir la masse avec les principes actifs mentionnés ci dessus.
Les principes actifs présent sont de dérivation naturelle, sauf l'acide hyaluronique, et il en résulte qu'ils sont bien tolérés par l'organisme, quand ils sont utilisés au niveau local, en conformité à la présente façon d'application.
En outre on pourrait même imaginer utiliser la présente préparation pour le soin du prurit, des brûlures, de l'érythème et des lésions dermoépidermiques de la muqueuse vaginale, provoqués par bactéries, protozaires ou mycètes, en association à d' autres remèdes pharmaceutiques spécifiques pour le soin de ces affections.

## Revendications

1. Préparation sous forme d'ovule vaginal constitué de glycérides semi-synthétiques apte à être introduit dans le vagin, **caractérisée par** une association des principes actifs : Acide Hyaluronique, Aloès Vera, Bevilaqua Hydrocotyle Asiatique, Souci Officinal, Huile de Cajeputier, pour le soin des états atrophiques et dystrophiques cervico-vaginaux (vaginites dues à carence d'oestrogènes,vaginites séniles, craurosis vulvo-vaginal), retard de cicatrisation cervico-vaginale du post-partum, en chirurgie gynécologique, dystrophies conséquentes à chimiothérapie ou radiations-ionisantes.

2. Préparation selon la revendication 1, **caractérisée en ce que** pour la production de la susdite préparation on utilise, pour ovules ayant un poids compris entre 2 et 5 g., une quantité d'acide hyaluronique comprise entre 5 et 100 mg.

3. Préparation selon l'une des revendications précédentes, **caractérisée en ce que** pour la production de la susdite préparation on utilise, pour les ovules ayant un poids compris entre 2 et 5 g., une quantité d'Aloès Vera comprise entre 20 et 200mg.

4. Préparation selon l'une des revendications précédentes, **caractérisée en ce que** pour la production de la susdite préparation on utilise, pour les ovules ayant un poids entre 2 et 5 g., une quantité de Bevilaqua Hydrocotyle Asiatique comprise entre 20 et 200 mg.

5. Préparation selon l'une des revendications précédentes, **caractérisée en ce que** pour la production de la susdite préparation on utilise, pour les ovules ayant un poids entre 2 et 5 g., une quantité de Souci Officinal comprise entre 20 et 200 mg.

6. Préparation selon l'une des revendications précédentes, **caractérisée en ce que** pour la production de la susdite préparation on utilise, pour les ovules ayant un poids entre 2 et 5 g., une quantité d'huile de Cajeputier comprise entre 1 et 10 mg.

## Claims

1. Preparation in the form of vaginal ovules consisting of semi-synthetic glycerides suited to be introduced into the vagina, **characterised by** an association of active principles: Hyaluronic Acid, Aloe Vera, Centella Asiatica, Calendula, Tea tree oil, for the treatment of cervicovaginal atrophic and dystrophic states (vaginitis due to oestrogen deficiency, senile vaginitis, vulvovaginal kraurosis), delay in the vulvovaginal tissue repairing process in post-partum, in gynaecological surgery, in the dystrophies following chemotherapy or ionising radiations.

2. Preparation according to claim 1, **characterized in that** for the production of the above-mentioned preparation, for ovules with a weight of between 2 and 5 g, a quantity of hyaluronic acid of between 5 and 100 mg is used.

3. Preparation according to any of the preceding claims, **characterized in that** for the production of the above-mentioned preparation, for ovules with a weight of between 2 and 5 g, a quantity of Aloe Vera of between 20 and 200 mg is used.

4. Preparation according to any of the preceding claims, **characterized in that** for the production of the above-mentioned preparation, for ovules with a weight of between 2 and 5 g, a quantity of Centella Asiatica of between 20 and 200 mg is used.

5. Preparation according to any of the preceding claims, **characterized in that** for the production of the above-mentioned preparation, for ovules with a weight of between 2 and 5 g, a quantity of Calendula of between 20 and 200 mg is used.

6. Preparation according to any of the preceding claims, **characterized in that** for the production of the above-mentioned preparation, for ovules with a weight of between 2 and 5 g, a quantity of Tea Tree Oil of between 1 and 10 mg is used.

## Patentansprüche

1. Präparat in Form von Vaginal Eizelle, Kombination aus halbsynthetischen Glitzeriden für die Einführung in die Vagina, zusammengesetzt aus einer Verbindung von folgenden Wirkstoffen: Hyaluron Säure, Aloe Vera, Hydrocotyle Asiatika, Calendula (Ringelblume), Teebaumöl. Zur Behandlung von atrophischen und distrophischen zervikal-vaginal Zuständen (Vaginitis hervorgerufen durch Östrogenmangel, senile Kolpitis, Kraurosis der Vulva), Verzögerung der Narbenbildung im Zervical-Vaginal-Bereich im Post-Partum. Desweiteren zur Behandlung in der gynäkologischen Chirurgie, bei Dystrophien im Anschluss an Chemotherapien und ionisierende Bestrahlungen.

2. Präparat gemäss Patentanspruch 1. Zur Herstellung des oben genannten Präparates, bestimmt für Eizellen mit einem Gewicht zwischen 2 und 5 g., wird eine Menge zwischen 5 und 100 mg von Hyaluron Säure benutzt.

3. Präparat gemäß der vorherigen Patentansprüche. Zur Herstellung des oben genannten Präparates, bestimmt für Eizellen mit einem Gewicht zwischen 2 und 5 g., wird eine Menge zwischen 20 und 200 mg von Aloe Vera Säure benutzt.

4. Präparat gemäß der vorherigen Patentansprüche. Zur Herstellung des oben genannten Präparates, bestimmt für Eizellen mit einem Gewicht zwischen 2 und 5 g., wird eine Menge zwischen 20 und 200 mg von Hydrocotyle Asiatika benutzt.

5. Präparat gemäß der vorherigen Patentansprüche. Zur Herstellung des oben genannten Präparates, bestimmt für Eizellen mit einem Gewicht zwischen 2 und 5 g., wird eine Menge zwischen 20 und 200 mg von Calendula (Ringelblume) benutzt.

6. Präparat gemäß der vorherigen Patentansprüche. Zur Herstellung des oben genannten Präparates, bestimmt für Eizellen mit einem Gewicht zwischen 2 und 5 g., wird eine Menge zwischen 1 und 10 mg von Teebaumöl benutzt.
